# EUROPEAN PATENT APPLICATION

(11) **EP 2 053 044 A1**
(43) Date of publication of application: **29.04.2009**
(21) Application number: 07020978.8
(22) Date of filing: 26.10.2007
(51) Int. Cl.: C07D 233/54, C07D 233/61, C07D 233/68, C07D 409/04, C07D 409/14, A01N 43/50

(54) **Novel imidazole derivatives**

(71) Applicant: Syngenta Participations AG, 4058 Basel (CH)
(72) Inventor: The designation of the inventor has not yet been filed
(74) Representative: Arunasalam, Velautha-Cumaran

(57) **Abstract**

The present invention relates to novel imidazole derivatives of formula I as active ingredients which have microbiocidal activity, in particular fungicidal activity: 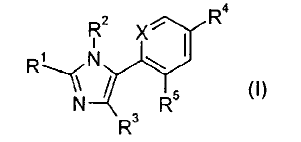 wherein
R¹ is halogen, C₁-C₄alkyl or C₁-C₄haloalkyl;
R² is an optionally substituted aryl or heteroaryl;
R³ is halogen;
R⁴ is hydrogen, halogen, C₁-C₄alkyl, C₁-C₄haloalkyl, C₁-C₄alkoxy. C₁-C₄haloalkoxy or cyano;
R⁵ is halogen;
X is N or C(R); and
R is hydrogen, halogen, C₁-C₄alkyl, C₁-C₄haloalkyl, C₁-C₄alkoxy, C₁-C₄haloalkoxy or cyano; or an agrochemically usable salt form thereof;

provided that
when X is C(R), R² cannot be an optionally substituted aryl.

## Description

The present invention relates to novel imidazole derivatives as active ingredients which have microbiocidal activity, in particular fungicidal activity. The invention also relates to preparation of these active ingredients, to novel heterocyclic derivatives used as intermediates in the preparation of these active ingredients, to preparation of these novel intermediates, to agrochemical compositions which comprise at least one of the novel active ingredients, to preparation of these compositions and to use of the active ingredients or compositions in agriculture or horticulture for controlling or preventing infestation of plants, harvested food crops, seeds or non-living materials by phytopathogenic microorganisms, preferably fungi.

The present invention provides a compound of formula I: 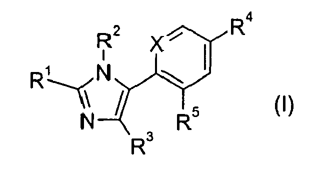 wherein
R¹ is halogen, C₁-C₄alkyl or C₁-C₄haloalkyl;
R² is an optionally substituted aryl or heteroaryl;
R³ is halogen;
R⁴ is hydrogen, halogen, C₁-C₄alkyl, C₁-C₄haloalkyl, C₁-C₄alkoxy, C₁-C₄haloalkoxy or cyano;
R⁵ is halogen;
X is N or C(R); and
R is hydrogen, halogen, C₁-C₄alkyl, C₁-C₄haloalkyl, C₁-C₄alkoxy, C₁-C₄haloalkoxy or cyano; or an agrochemically usable salt form thereof;
   provided that
   when X is C(R), R² cannot be an optionally substituted aryl.

In the above definition aryl includes aromatic hydrocarbon rings like phenyl, naphthyl, anthracenyl, phenanthrenyl and biphenyl, with phenyl being preferred.

Heteroaryl stands for aromatic ring systems comprising mono-, bi- or tricyclic systems wherein at least one oxygen, nitrogen or sulfur atom is present as a ring member. Examples are furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyridazinyl, pyrimidinyl, pyrazinyl, triazinyl, tetrazinyl, indolyl, benzothiophenyl, benzofuranyl, benzimidazolyl, indazolyl, benzotriazolyl, benzothiazolyl, benzoxazolyl, quinolyl, isoquinolyl, phthalazinyl, quinoxalinyl, quinazolinyl, cinnolinyl and naphthyridinyl.

The above or below mentioned fused ring, carbocyclic ring, heterocyclic ring, aryl group and heteroaryl group may be optionally substituted. This means that they may carry one or more identical or different substituents. Normally not more than three substituents are present at the same time. Examples of substituents are: halogen, alkyl, haloalkyl, cycloalkyl, cycloalkylalkyl, alkenyl, haloalkenyl, cycloalkenyl, alkynyl, haloalkynyl, alkyloxy, haloalkyloxy, cycloalkoxy, alkenyloxy, haloalkenyloxy, alkynyloxy, haloalkenyloxy, alkylthio, haloalkylthio, cycloalkylthio, alkenylthio, alkynylthio, alkylcarbonyl, haloalkylcarbonyl, cycloalkylcarbonyl, alkenylcarbonyl, alkynylcarbonyl, alkoxyalkyl, cyano, nitro, hydroxy, mercapto, amino, alkylamino, dialkylamino. Typical examples for optionally substituted aryl include 2-fluorophenyl, 3-fluorophenyl, 4-fluorophenyl, 2-chlorophenyl, 3-chlorophenyl, 4-chlorophenyl, 3-bromophenyl, 4-bromophenyl, m-tolyl, p-tolyl, 3-trifluoromethylphenyl, 4-trifluoromethylphenyl, 3-methoxyphenyl, 4-methoxyphenyl, 3-trifluoromethoxyphenyl, 4-trifluoromethoxyphenyl, 3-cyanophenyl, 4-cyanophenyl, 2,4-difluorophenyl, 2,5-difluorophenyl, 2,6-difluorophenyl, 3,4-difluorophenyl, 2,4-dichlorophenyl, 2,5-dichlorophenyl, 2,6-dichlorophenyl, 3,4-dichlorophenyl, 3,4-dimethylphenyl, 3,4-dimethoxyphenyl, 2-chloro-4-fluorophenyl, 2-chloro-5-fluorophenyl, 2-chloro-6-fluorophenyl, 3-chloro-4-fluorophenyl, 3-chloro-6-fluorophenyl, 3-chloro-4-methylphenyl, 3-chloro-4-methoxyphenyl, 4-chloro-2-fluorophenyl, 4-chloro-3-fluorophenyl, 4-chloro-3-methylphenyl, 4-chloro-3-methoxyphenyl, 3-fluoro-4-methoxyphenyl, 3-fluoro-4-methylphenyl, 4-fluoro-3-methoxyphenyl, 4-fluoro-3-methylphenyl, 3-methoxy-4-methylphenyl, 4-methoxy-3-methylphenyl, 2,6-difluoro-4-methylphenyl, 2,6-difluoro-4-trifluoromethylphenyl, 2,6-difluoro-4-methoxyphenyl, 2,6-difluoro-4-trifluoromethoxyphenyl, 2,6-difluoro-4-cyanophenyl, 2,4,6-trifluorophenyl, 2,5,6-trifluorophenyl. Typical examples for optionally substituted heteroaryl include 6-chloropyridin-2-yl, 6-fluoropyridin-2-yl, 6-methoxypyridin-2-yl, 6-methylpyridin-2-yl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-methoxypyridin-4-yl, 2-methylpyridin-4-yl, 3,5-dichloropyridin-2-yl, 3,5-difluoropyridin-2-yl, 3-chloro-5-fluoropyridin-2-yl, 3-chloro-5-methylpyridin-2-yl, 3-chloro-5-trifluoromethylpyridin-2-yl, 3-chloro-5-methoxypyridin-2-yl, 3-chloro-5-trifluoromethoxypyridin-2-yl, 3-chloro-5-cyanopyridin-2-yl, 5-chloro-3-fluoropyridin-2-yl, 3-fluoro-5-methylpyridin-2-yl, 3-fluoro-5-trifluoromethylpyridin-2-yl, 3-fluoro-5-methoxypyridin-2-yl, 3-fluoro-5-trifluoromethoxypyridin-2-yl, 3-fluoro-5-cyanopyridin-2-yl, 5-chlorothiophen-2-yl, 5-bromothiophen-2-yl, 5-methoxythiophen-2-yl, 4-methoxyquinolin-2-yl, 4-methylquinolin-2-yl.

In the above definition halogen is fluorine, chlorine, bromine or iodine.

The alkyl, alkenyl or alkynyl radicals may be straight-chained or branched.

Alkyl on its own or as part of another substituent is, depending upon the number of carbon atoms mentioned, for example, methyl, ethyl, propyl, butyl, pentyl, hexyl and the isomers thereof, for example, isopropyl, isobutyl, sec-butyl, tert-butyl, isopentyl or tert-pentyl.

A haloalkyl group may contain one or more identical or different halogen atoms and, for example, may stand for CH₂Cl, CHCl₂, CCl₃, CH₂F, CHF₂, CF₃, CF₃CH₂, CH₃CF₂, CF₃CF₂ or CCl₃CCl₂.

Cycloalkyl on its own or as part of another substituent is, depending upon the number of carbon atoms mentioned, for example, cyclopropyl, cyclobutyl, cyclopentyl or cyclohexyl.

Alkenyl on its own or as part of another substituent is, depending upon the number of carbon atoms mentioned, for example, ethenyl, allyl, 1-propenyl, buten-2-yl, buten-3-yl, penten-1-yl, penten-3-yl, hexen-1-yl or 4-methyl-3-pentenyl.

Alkynyl on its own or as part of another substituent is, depending upon the number of carbon atoms mentioned, for example, ethynyl, propyn-1-yl, propyn-2-yl, butyn-1-yl, butyn-2-yl, 1-methyl-2-butynyl, hexyn-1-yl or 1-ethyl-2-butynyl.

The presence of one or more possible asymmetric carbon atoms in a compound of formula I means that the compounds may occur in optically isomeric, that means enantiomeric or diastereomeric forms. As a result of the presence of a possible aliphatic C=C double bond, geometric isomerism, that means cis-trans or (*E*)-(*Z*) isomerism may also occur. Also atropisomers may occur as a result of restricted rotation about a single bond. Formula I is intended to include all those possible isomeric forms and mixtures thereof. The present invention intends to include all those possible isomeric forms and mixtures thereof for a compound of formula I.

In each case, the compounds of formula I according to the invention are in free form or in an agronomically usable salt form.

In a first embodiment, compounds of formula I according to the invention have R¹ which is halogen or C₁-C₄alkyl.

In a second embodiment, compounds of formula I according to the invention have R² which is an optionally substituted phenyl, naphthyl, thienyl, pyridyl or quinolyl.

In a third embodiment, compounds of formula I according to the invention have R³ which is fluoro, chloro or bromo.

In a fourth embodiment, compounds of formula I according to the invention have R⁴ which is hydrogen, halogen, C₁-C₃alkyl, C₁-C₃haloalkyl, C₁-C₃alkoxy or C₁-C₃haloalkoxy or cyano.

In a fifth embodiment, compounds of formula I according to the invention have R⁵ which is fluoro, chloro, bromo or iodo.

In a sixth embodiment, compounds of formula I according to the invention have X which is N, C(H), C(halogen), C(C₁-C₄alkyl), C(C₁-C₄haloalkyl), C(C₁-C₄alkoxy) or C(C₁-C₄haloalkoxy).

Preferred subgroups of compounds of formula I according to the invention are those wherein R¹ is fluoro, chloro, bromo, iodo, C₁-C₂alkyl or C₁-C₂haloalkyl;
R² is an optionally substituted phenyl, pyridyl, thienyl or quinolyl;
R³ is fluoro, chloro or bromo;
R⁴ is hydrogen, fluoro, chloro, bromo, C₁-C₃alkyl, C₁-C₃haloalkyl, C₁-C₃alkoxy or C₁-C₃haloalkoxy or cyano;
R⁵ is fluoro, chloro or bromo; and
X is N, C(H), C(Cl), C(F), C(Br), C(I), C(C₁-C₃alkyl), C(C₁-C₃haloalkyl), C(C₁-C₃alkoxy) or C(C₁-C₃haloalkoxy).

More preferred subgroups of compounds of formula I according to the invention are those wherein
R¹ is fluoro, chloro, bromo, methyl or ethyl;
R² is phenyl, 3-fluorophenyl, 4-fluorophenyl, 3-chlorophenyl, 4-chlorophenyl, 3-bromophenyl, 4-bromophenyl, m-tolyl, p-tolyl, 3-trifluoromethylphenyl, 4-trifluoromethylphenyl, 3-methoxyphenyl, 4-methoxyphenyl, 3-trifluoromethoxyphenyl, 4-trifluoromethoxyphenyl, 3-cyanophenyl, 4-cyanophenyl, 3,4-difluorophenyl, 3,4-dichlorophenyl, 3,4-dimethylphenyl, 3,4-dimethoxyphenyl, 3-chloro-4-fluorophenyl, 3-chloro-4-methylphenyl, 3-chloro-4-methoxyphenyl, 4-chloro-3-fluorophenyl, 4-chloro-3-methylphenyl, 4-chloro-3-methoxyphenyl, naphth-2-yl, 3-fluoro-4-methoxyphenyl, 3-fluoro-4-methylphenyl, 4-fluoro-3-methoxyphenyl, 4-fluoro-3-methylphenyl, 3-methoxy-4-methylphenyl, 4-methoxy-3-methylphenyl, pyridin-2-yl, pyridin-3-yl, pyridin-4-yl, 6-chloropyridin-2-yl, 6-fluoropyridin-2-yl, 6-methoxypyridin-2-yl, 6-methylpyridin-2-yl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-methoxypyridin-4-yl, 2-methylpyridin-4-yl, 5-chlorothiophen-2-yl, 5-bromothiophen-2-yl, 5-methoxythiophen-2-yl, quinolin-2-yl, quinolin-3-yl, 4-methoxyquinolin-2-yl or 4-methylquinolin-2-yl;
R³ is fluoro or chloro;
R⁴ is hydrogen, fluoro, chloro, C₁-C₂alkyl, C₁-C₂haloalkyl, C₁-C₂alkoxy, C₁-C₂haloalkoxy or cyano;
R⁵ is fluoro or chloro; and
X is N, C(H), C(Cl), C(F), C(Br), C(C₁-C₂alkyl), C(C₁-C₂haloalkyl), C(C₁-C₂alkoxy) or C(C₁-C₂haloalkoxy).

Most preferred subgroups of compounds of formula I according to the invention are those wherein
R¹ is fluoro, chloro, methyl or ethyl;
R² is 4-fluorophenyl, 4-chlorophenyl, 4-bromophenyl, p-tolyl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-methoxypyridin-4-yl, 2-methylpyridin-4-yl, quinolin-2-yl, quinolin-3-yl, 4-methoxyquinolin-2-yl or 4-methylquinolin-2-yl;
R³ is fluoro or chloro;
R⁴ is hydrogen, fluoro, chloro, C₁-C₂alkyl, C₁-C₂haloalkyl or C₁-C₂alkoxy;
R⁵ is fluoro or chloro; and
X is N, C(H), C(Cl) or C(F).

Especially preferred subgroups of compounds of formula I according to the invention are those wherein
R¹ is chloro or methyl;
R² is 6-chloropyridin-3-yl, 6-methylpyridin-3-yl or quinolin-3-yl;
R³ is chloro;
R⁴ is fluoro; and
R⁵ is fluoro; and
X is C(F).

Preferred individual compounds are:
2-chloro-5-[2,4-dichloro-5-(2,4,6-trifluoro-phenyl)-imidazol-1-yl]-pyridine;
2-chloro-5-[4-chloro-2-methyl-5-(2,4,6-trifluoro-phenyl)-imidazol-1-yl]-pyridine;
5-[4-chloro-2-methyl-5-(2,4,6-trifluoro-phenyl)-imidazol-1-yl]-2-methoxy-pyridine;
2-[4-chloro-2-methyl-5-(2,4,6-trifluoro-phenyl)-imidazol-1-yl]-quinoline;
2-[4-chloro-2-methyl-5-(2,4,6-trifluoro-phenyl)-imidazol-1-yl]-4-methoxy-quinoline;
3-[4-chloro-2-methyl-5-(2,4,6-trifluoro-phenyl)-imidazol-1-yl]-quinoline;
3-[2,4-dichloro-5-(2,4,6-trifluoro-phenyl)-imidazol-1-yl]-q uinoline;
3-[2,4-dichloro-5-(2,6-difluoro-4-methoxy-phenyl)-imidazol-1-yl]-quinoline;
2-[4-chloro-5-(2,6-difluoro-4-methoxy-phenyl)-2-methyl-imidazol-1-yl]-4-methoxy-quinoline;
3-[4-chloro-5-(2,6-difluoro-4-methoxy-phenyl)-2-methyl-imidazol-1-yl]-quinoline;
2-chloro-5-[4-chloro-5-(2,6-difluoro-4-methoxy-phenyl)-2-methyl-imidazol-1-yl]-pyridine;
3,5-dichloro-2-[5-chloro-3-(6-chloro-pyridin-3-yl)-2-methyl-3H-imidazol-4-yl]-pyridine; and
2-[4-chloro-5-(3,5-dichloro-pyridin-2-yl)-2-methyl-imidazol-1-yl]-quinoline.

Compounds of formula (I.1), 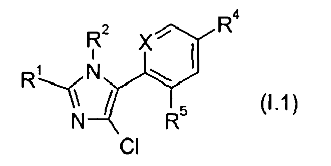 in which R¹, R², R⁴, R⁵ and X have the meanings given above, are examples of compounds of general formula (I) and can be made as shown in the following schemes.

The compounds of formula I.1, wherein R², R⁴, R⁵ and X are as defined for compound of formula I, and R¹ is C₁-C₄alkyl or C₁-C₄haloalkyl, can be obtained by reaction of a compound of formula II, wherein R², R⁴, R⁵ and X are as defined for compound of formula I, and R¹ is C₁-C₄alkyl or C₁-C₄haloalkyl, with N-chlorosuccinimide (NCS) or molecular chlorine.

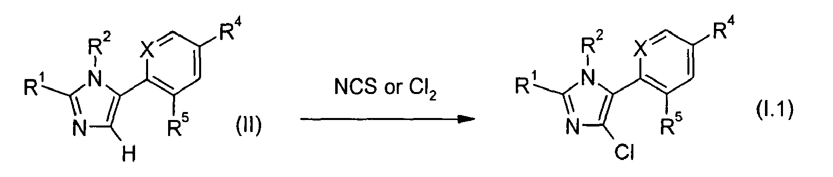

The compounds of formula II, wherein R², R⁴, R⁵ and X are as defined for compound of formula I, and R¹ is C₁-C₄alkyl, can be obtained by transformation of a compound of formula III, wherein R², R⁴, R⁵ and X are as defined for compound of formula I, with a reagent of formula (R¹)₃Al, wherein R¹ is C₁-C₄alkyl, preferably methyl, in the presence of a transition metal catalyst.

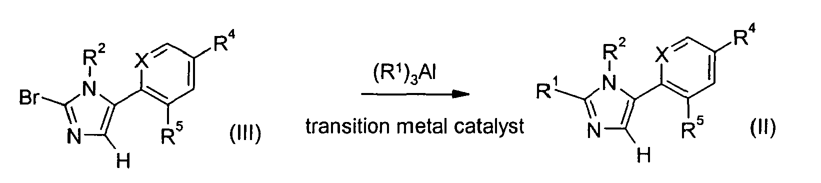

The compounds of formula II, wherein R², R⁴, R⁵ and X are as defined for compound of formula I, and R¹ is C₁-C₄alkyl or C₁-C₄haloalkyl, can alternatively be obtained by transformation of a compound of formula IV, wherein R², R⁴, R⁵ and X are as defined for compound of formula I, with a strong base, e.g. lithium di-isopropylamide, followed by a reagent of formula R¹Hal, wherein R¹ is C₁-C₄alkyl or C₁-C₄haloalkyl, and Hal is halogen, preferably bromine or iodine.

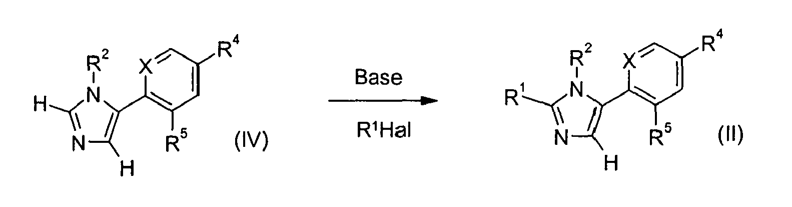

The compounds of formula I, wherein R², R³, R⁴, R⁵ and X are as defined for compound of formula I, and R¹ is Halogen, preferably chlorine or bromine, can be obtained by reaction of a compound of formula IV, wherein R², R⁴, R⁵ and X are as defined for compound of formula I, with at least 2 equivalents of N-chlorosuccinimide (NCS), N-bromosuccinimide (NBS), molecular chlorine or bromine.

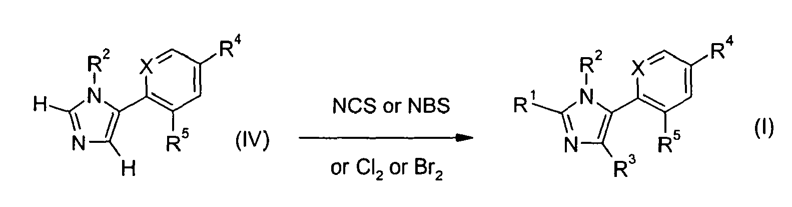

The compounds of formula III, wherein R², R⁴, R⁵ and X are as defined for compound of formula I, can be obtained by transformation of a compound of formula IV, wherein R², R⁴, R⁵ and X are as defined for compound of formula I, with N-bromosuccinimide (NBS).

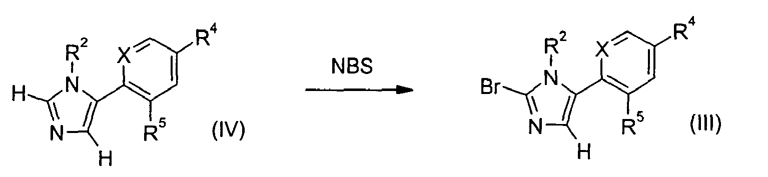

The compounds of formula IV, wherein R², R⁴, R⁵ and X are as defined for compound of formula I, can be obtained by reaction of a compound of formula V, wherein R²,, R⁴, R⁵ and X are as defined for compound of formula I, with toluenesulfonylmethyl isocyanide (TosMIC) in the presence of a base, e.g. anhydrous potassium carbonate, as already described in Journal of Medicinal Chemistry 2003, 46, 3463.

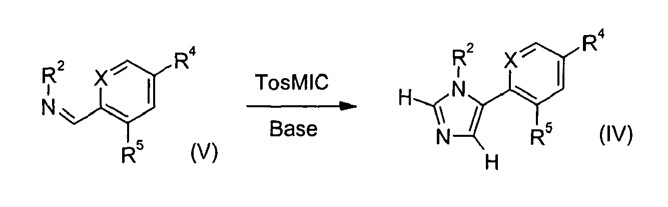

The compounds of formula V, wherein R², R⁴, R⁵ and X are as defined for compound of formula I, can be obtained by reaction of an aldehyde of formula VI, wherein, R⁴, R⁵ and X are as defined for compound of formula I, with an amine of formula VII, wherein R² is as defined for compound of formula I, as already described in Journal of Medicinal Chemistry 2003,46,3463.

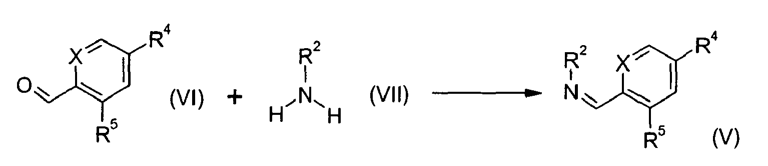

Surprisingly, it has now been found that the novel compounds of formula I have, for practical purposes, a very advantageous level of biological activity for protecting plants against diseases that are caused by fungi as well as by bacteria and viruses.

The compounds of formula I can be used in unmodified form or, preferably, together with carriers and adjuvants conventionally employed in the art of formulation.

Therefore the invention also relates to compositions for controlling and protecting against phytopathogenic micro-organisms, comprising a compound of formula I and an inert carrier, and to a method of controlling or preventing infestation of useful plants by phytopathogenic micro-organisms, wherein a composition, comprising a compound of formula I as active ingredient and an inert carrier, is applied to the plants, to parts thereof or the locus thereof.

In addition, the invention could be used to protect non-living materials from fungal attack, e.g. lumber, wall boards and paint.

To this end compounds of formula I and inert carriers are conveniently formulated in known manner to mollifiable concentrates, coat able pastes, directly spray able or dilatable solutions, dilute emulsions, wet table powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or pacifiers as well as fertilizers, micronutrient donors or other formulations for obtaining special effects.

Suitable carriers and adjuvants can be solid or liquid and are substances useful in formulation technology, e.g. natural or regenerated mineral substances, solvents, dispersants, wetting agents, tackifiers, thickeners, binders or fertilizers. Such carriers are for example described in WO 97/33890.

The compounds of formula I or compositions, comprising a compound of formula I as active ingredient and an inert carrier, can be applied to the locus of the plant or plant to be treated, simultaneously or in succession with further compounds. These further compounds can be e.g. fertilizers or micronutrient donors or other preparations which influence the growth of plants. They can also be selective herbicides as well as insecticides, fungicides, bactericides, nematicides, molluscicides or mixtures of several of these preparations, if desired together with further carriers, surfactants or application promoting adjuvants customarily employed in the art of formulation.

A preferred method of applying a compound of formula I, or a composition, comprising a compound of formula I as active ingredient and an inert carrier, is foliar application. The frequency of application and the rate of application will depend on the risk of infestation by the corresponding pathogen. However, the compounds of formula I can also penetrate the plant through the roots via the soil (systemic action) by drenching the locus of the plant with a liquid formulation, or by applying the compounds in solid form to the soil, e.g. in granular form (soil application). In crops of water rice such granulates can be applied to the flooded rice field. The compounds of formula I may also be applied to seeds (coating) by impregnating the seeds or tubers either with a liquid formulation of the fungicide or coating them with a solid formulation.

A formulation, i.e. a composition comprising the compound of formula I and, if desired, a solid or liquid adjuvant, is prepared in a known manner, typically by intimately mixing and/or grinding the compound with extenders, for example solvents, solid carriers and, optionally, surface-active compounds (surfactants).

The agrochemical formulations will usually contain from 0.1 to 99% by weight, preferably from 0.1 to 95% by weight, of the compound of formula I, 99.9 to 1 % by weight, preferably 99.8 to 5% by weight, of a solid or liquid adjuvant, and from 0 to 25% by weight, preferably from 0.1 to 25% by weight, of a surfactant.

Whereas it is preferred to formulate commercial products as concentrates, the end user will normally use dilute formulations.

Advantageous rates of application are normally from 5g to 2kg of active ingredient (a.i.) per hectare (ha), preferably from 10g to 1 kg a.i./ha, most preferably from 20g to 600g a.i./ha. When used as seed drenching agent, convenient rates of application are from 10mg to 1g of active substance per kg of seeds. The rate of application for the desired action can be determined by experiments. It depends for example on the type of action, the developmental stage of the useful plant, and on the application (location, timing, application method) and can, owing to these parameters, vary within wide limits.

The invention relates to a method of controlling or preventing infestation of useful plants by phytopathogenic micro-organisms, wherein a compound of formula I is applied as active ingredient to the plants, to parts thereof or the locus thereof. The compounds of formula I according to the invention are distinguished by excellent activity at low rates of application, by being well tolerated by plants and by being environmentally safe. They have very useful curative, preventive and systemic properties and are used for protecting numerous useful plants. The compounds of formula I can be used to inhibit or destroy the diseases that occur on plants or parts of plants (fruit, blossoms, leaves, stems, tubers, roots) of different crops of useful plants, while at the same time protecting also those parts of the plants that grow later e.g. from phytopathogenic micro-organisms.

It is also possible to use compounds of formula I as dressing agents for the treatment of plant propagation material, in particular of seeds (fruit, tubers, grains) and plant cuttings (e.g. rice), for the protection against fungal infections as well as against phytopathogenic fungi occurring in the soil.

Furthermore the compounds of formula I according to the invention may be used for controlling fungi in related areas, for example in the protection of technical materials, including wood and wood related technical products, in food storage or in hygiene management.

Within the scope of the invention, useful plants to be protected typically comprise the following species of plants: cereal (wheat, barley, rye, oat, rice, maize, sorghum and related species); beet (sugar beet and fodder beet); pomes, drupes and soft fruit (apples, pears, plums, peaches, almonds, cherries, strawberries, raspberries and blackberries); leguminous plants (beans, lentils, peas, soybeans); oil plants (rape, mustard, poppy, olives, sunflowers, coconut, castor oil plants, cocoa beans, groundnuts); cucumber plants (pumpkins, cucumbers, melons); fibre plants (cotton, flax, hemp, jute); citrus fruit (oranges, lemons, grapefruit, mandarins); vegetables (spinach, lettuce, asparagus, cabbages, carrots, onions, tomatoes, potatoes, paprika); lauraceae (avocado, cinnamomum, camphor) or plants such as tobacco, nuts, coffee, eggplants, sugar cane, tea, pepper, vines, hops, bananas and natural rubber plants, as well as ornamentals.

The term "useful plants" is to be understood as including also useful plants that have been rendered tolerant to herbicides like bromoxynil or classes of herbicides (such as, for example, HPPD inhibitors, ALS inhibitors, for example primisulfuron, prosulfuron and trifloxysulfuron, EPSPS (5-enol-pyrovyl-shikimate-3-phosphate-synthase) inhibitors, GS (glutamine synthetase) inhibitors or PPO (protoporphyrinogen-oxidase) inhibitors) as a result of conventional methods of breeding or genetic engineering. An example of a crop that has been rendered tolerant to imidazolinones, e.g. imazamox, by conventional methods of breeding (mutagenesis) is Clearfield® summer rape (Canola). Examples of crops that have been rendered tolerant to herbicides or classes of herbicides by genetic engineering methods include glyphosate- and glufosinate-resistant maize varieties commercially available under the trade names RoundupReady® , Herculex I® and LibertyLink®.

The term "useful plants" is to be understood as including also useful plants which have been so transformed by the use of recombinant DNA techniques that they are capable of synthesising one or more selectively acting toxins, such as are known, for example, from toxin-producing bacteria, especially those of the genus Bacillus.

The term "useful plants" is to be understood as including also useful plants which have been so transformed by the use of recombinant DNA techniques that they are capable of synthesising antipathogenic substances having a selective action, such as, for example, the so-called "pathogenesis-related proteins" (PRPs, see e.g. EP-A-0 392 225). Examples of such antipathogenic substances and transgenic plants capable of synthesising such antipathogenic substances are known, for example, from EP-A-0 392 225, WO 95/33818, and EP-A-0 353 191. The methods of producing such transgenic plants are generally known to the person skilled in the art and are described, for example, in the publications mentioned above.

The term "locus" of a useful plant as used herein is intended to embrace the place on which the useful plants are growing, where the plant propagation materials of the useful plants are sown or where the plant propagation materials of the useful plants will be placed into the soil. An example for such a locus is a field, on which crop plants are growing.

The term "plant propagation material" is understood to denote generative parts of the plant, such as seeds, which can be used for the multiplication of the latter, and vegetative material, such as cuttings or tubers, for example potatoes. There may be mentioned for example seeds (in the strict sense), roots, fruits, tubers, bulbs, rhizomes and parts of plants. Germinated plants and young plants which are to be transplanted after germination or after emergence from the soil, may also be mentioned. These young plants may be protected before transplantation by a total or partial treatment by immersion. Preferably "plant propagation material" is understood to denote seeds.

The compounds of formula I are, for example, effective against the phytopathogenic fungi of the following classes: Fungi imperfecti (e.g. Botrytis, Pyricularia, Helminthosporium, Fusarium, Septoria, Cercospora and Alternaria) and Basidiomycetes (e.g. Rhizoctonia, Hemileia, Puccinia). Additionally, they are also effective against the Ascomycetes classes (e.g. Venturia and Erysiphe, Podosphaera, Monilinia, Uncinula) and of the Oomycetes classes (e.g. Phytophthora, Pythium, Plasmopara). Outstanding activity has been observed against powdery mildew diseases (e.g. Uncinula necator). Furthermore, the novel compounds of formula I are effective against phytopathogenic bacteria and viruses (e.g. against Xanthomonas spp, Pseudomonas spp, Erwinia amylovora as well as against the tobacco mosaic virus). Good activity has been observed against Asian soybean rust (Phakopsora pachyrhizi).

Surprisingly, it has now been found that the compounds of formula I, or a pharmaceutical salt thereof, described above have also an advantageous spectrum of activity for the treatment and/or prevention of microbial infection in an animal.

"Animal" can be any animal, for example, insect, mammal, reptile, fish, amphibian, preferably mammal, most preferably human. "Treatment" means the use on an animal which has microbial infection in order to reduce or slow or stop the increase or spread of the infection, or to reduce the infection or to cure the infection. "Prevention" means the use on an animal which has no apparent signs of microbial infection in order to prevent any future infection, or to reduce or slow the increase or spread of any future infection.

According to the present invention there is provided the use of a compound of formula I in the manufacture of a medicament for use in the treatment and/or prevention of microbial infection in an animal. There is also provided the use of a compound of formula I as a pharmaceutical agent. There is also provided the use of a compound of formula I as an antimicrobial agent in the treatment of an animal. According to the present invention there is also provided a pharmaceutical composition comprising as an active ingredient a compound of formula I, or a pharmaceutically acceptable salt thereof, and a pharmaceutically acceptable diluent or carrier. This composition can be used for the treatment and/or prevention of antimicrobial infection in an animal. This pharmaceutical composition can be in a form suitable for oral administration, such as tablet, lozenges, hard capsules, aqueous suspensions, oily suspensions, emulsions dispersible powders, dispersible granules, syrups and elixirs. Alternatively this pharmaceutical composition can be in a form suitable for topical application, such as a spray, a cream or lotion. Alternatively this pharmaceutical composition can be in a form suitable for parenteral administration, for example injection. Alternatively this pharmaceutical composition can be in inhalable form, such as an aerosol spray.

The compounds of formula I are effective against various microbial species able to cause a microbial infection in an animal. Examples of such microbial species are those causing Aspergillosis such as *Aspergillus fumigatus, A. flavus, A. terrus, A. nidulans* and *A. niger*, those causing Blastomycosis such as *Blastomyces dermatitidis*; those causing Candidiasis such as *Candida albicans, C. glabrata, C. tropicalis, C. parapsilosis, C. krusei* and *C. lusitaniae*; those causing Coccidioidomycosis such as *Coccidioides immitis*; those causing Cryptococcosis such as *Cryptococcus neoformans*; those causing Histoplasmosis such as *Histoplasma capsulatum* and those causing Zygomycosis such as *Absidia corymbifera, Rhizomucor pusillus* and *Rhizopus arrhizus*. Further examples are Fusarium Spp such as *Fusarium oxysporum* and *Fusarium solani* and Scedosporium Spp such as *Scedosporium apiospermum* and *Scedosporium prolificans*. Still further examples are Microsporum Spp, Trichophyton Spp, Epidermophyton Spp, Mucor Spp, Sporothorix Spp, Phialophora Spp, Cladosporium Spp, Petriellidium spp, Paracoccidioides Spp and Histoplasma Spp.

The compounds of formula I are normally used in the form of fungicidal compositions for controlling or protecting against phytopathogenic microorganisms, comprising as active ingredient at least one compound of formula I, in free form or in agrochemically usable salt form, and at least one of the above-mentioned adjuvants.

The compounds of formula I can be mixed with other fungicides, resulting in some cases in unexpected synergistic activities. Mixing components which are particularly preferred are:
Azoles, such as azaconazole, BAY 14120, bitertanol, bromuconazole, cyproconazole, difenoconazole, diniconazole, epoxiconazole, fenbuconazole, fluquinconazole, flusilazole, flutriafol, hexaconazole, imazalil, imibenconazole, ipconazole, metconazole, myclobutanil, pefurazoate, penconazole, prothioconazole, pyrifenox, prochloraz, propiconazole, simeconazole, tebuconazole, tetraconazole, triadimefon, triadimenol, triflumizole, triticonazole;
Pyrimidinyl carbinoles, such as ancymidol, fenarimol, nuarimol;
2-amino-pyrimidines, such as bupirimate, dimethirimol, ethirimol;
Morpholines, such as dodemorph, fenpropidine, fenpropimorph, spiroxamine, tridemorph;
Anilinopyrimidines, such as cyprodinil, mepanipyrim, pyrimethanil;
Pyrroles, such as fenpiclonil, fludioxonil;
Phenylamides, such as benalaxyl, furalaxyl, metalaxyl, R-metalaxyl, ofurace, oxadixyl;
Benzimidazoles, such as benomyl, carbendazim, debacarb, fuberidazole, thiabendazole;
Dicarboximides, such as chlozolinate, dichlozoline, iprodione, myclozoline, procymidone, vinclozoline;
Carboxamides, such as boscalid, carboxin, fenfuram, flutolanil, mepronil, oxycarboxin, penthiopyrad, thifluzamide; guanidines, such as guazatine, dodine, iminoctadine;
Strobilurines, such as azoxystrobin, dimoxystrobin, enestroburin, fluoxastrobin, kresoxim-methyl, metominostrobin, trifloxystrobin, orysastrobin, picoxystrobin, pyraclostrobin;
Dithiocarbamates, such as ferbam, mancozeb, maneb, metiram, propineb, thiram, zineb, ziram;
N-halomethylthiotetrahydrophthalimides, such as captafol, captan, dichlofluanid, fluoromides, folpet, tolyfluanid;
Cu-compounds, such as Bordeaux mixture, copper hydroxide, copper oxychloride, copper sulfate, cuprous oxide, mancopper, oxine-copper;
Nitrophenol-derivatives, such as dinocap, nitrothal-isopropyl;
Organo-phosphorus-derivatives, such as edifenphos, iprobenphos, isoprothiolane, phosdiphen, pyrazophos, tolclofos-methyl;
Pyridazine-derivatives which are known and may be prepared by methods as described in WO 05/121104, WO 06/001175 and WO 07/066601, such as 3-chloro-5-(4-chloro-phenyl)-6-methyl-4-(2,4,6-trifluoro-phenyl)-pyridazine (formula P.1), 3-chloro-6-methyl-5-p-tolyl-4-(2,4,6-trifluoro-phenyl)-pyridazine (formula P.2) and 3-chloro-4-(3-chloro-5-methoxy-pyridin-2-yl)-5-(4-chloro-phenyl)-6-methyl-pyridazine (formula P.3); 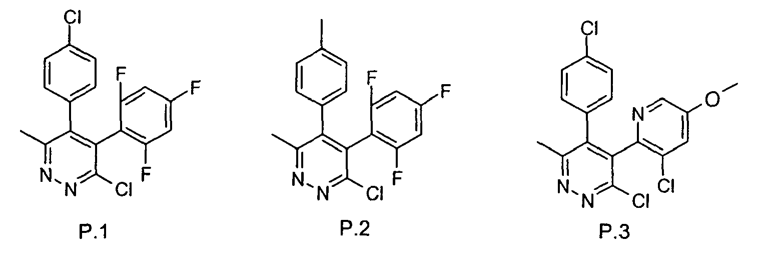
Triazolopyrimidine derivatives which are known and may be prepared by methods as described in WO98/46607, such as 5-chloro-7-(4-methyl-piperidin-1-yl)-6-(2,4,6-trifluorophenyl)- [1,2,4]triazolo[1,5-a]pyrimidine (formula T.1); 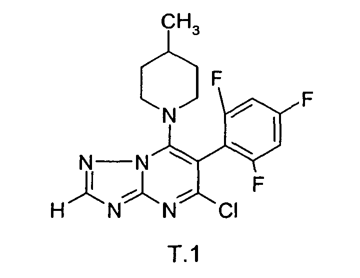
Carboxamide derivatives which are known and may be prepared by methods as described in WO04/035589, WO06/37632, WO03/074491 or WO03070705, such as 3-difluoromethyl-1-methyl-1 H-pyrazole-4-carboxylic acid (9-isopropyp-1,2,3,4-tetrahaydro-1,4-methano-naphthalen-5-yl)-amide (formula U.1), 3-difluoromethyl-1-methyl-1H-pyrazole-4-carboxylic acid (2-bicyclopropyl-2-yl-phenyl)-amide (formula U.2) or N-(3',4'-dichloro-5-fluoro-1,1'-biphenyl-2-yl)-3-(difluoromethyl)-1-methyl-1H-pyrazole-4-carboxamide; 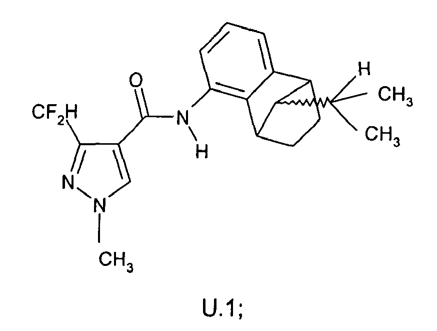 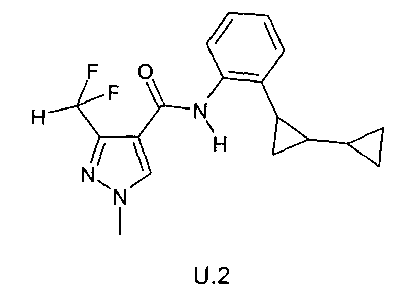
Benzamide derivatives which are known and may be prepared by methods as described in WO 2004/016088, such as N-{-2-[3-chloro-5-(trifluoromethyl)-2-pyridinyl]ethyl}-2-trifluoromethylbenzamide, which is also known under the name fluopyram (formula V.1); 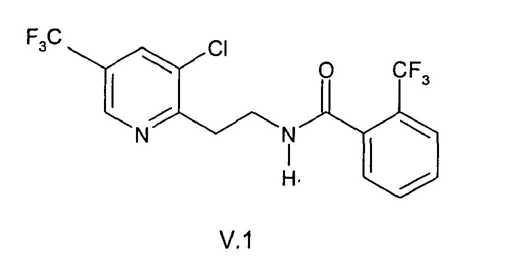 and
Various others, such as acibenzolar-S-methyl, anilazine, benthiavalicarb, blasticidin-S, chinomethionate, chloroneb, chlorothalonil, cyflufenamid, cymoxanil, dichlone, diclocymet, diclomezine, dicloran, diethofencarb, dimethomorph, flumorph, dithianon, ethaboxam, etridiazole, famoxadone, fenamidone, fenoxanil, fentin, ferimzone, fluazinam, fluopicolide, flusulfamide, fenhexamid, fosetyl-aluminium, hymexazol, iprovalicarb, cyazofamid, kasugamycin, mandipropamid, methasulfocarb, metrafenone, nicobifen, pencycuron, phthalide, polyoxins, probenazole, propamocarb, proquinazid, pyroquilon, quinoxyfen, quintozene, sulfur, tiadinil, triazoxide, tricyclazole, triforine, validamycin, zoxamide and glyphosate.

Another aspect of invention is related to the use of a compound of formula I, of a composition comprising at least one compound of formula I or of a fungicidal mixture comprising at least one compound of formula I in admixture with other fungicides, as described above, for controlling or preventing infestation of plants, harvested food crops or non-living materials by phytopathogenic microorganisms, preferably fungal organisms.

A further aspect of invention is related to a method of controlling or preventing an infestation of crop plants or of non-living materials by phytopathogenic or spoilage microorganisms or organisms potentially harmful to man, especially fungal organisms, which comprises the application of a compound of formula I as active ingredient to the plants, to parts of the plants or to the locus thereof, or to any part of the non-living materials. Controlling or preventing means reducing the infestation of crop plants or of non-living materials by phytopathogenic or spoilage microorganisms or organisms potentially harmful to man, especially fungal organisms, to such a level that an improvement is demonstrated.

The following non-limiting examples illustrate the above-described invention in more detail.

### Example 1: This example illustrates the preparation of 2-Chloro-5-[4-chloro-2-methyl-5-(2,4,6-trifluoro-phenyl)-imidazol-1-yl]-pyridine (Compound No.I.j.210)

### a) Preparation of (6-Chloro-pyridin-3-yl)-[1-(2,4,6-trifluoro-phenyl)-meth-(E)-ylidene]-amine

6-Chloro-pyridin-3-ylamine (5g) and 2,4,6-trifluoro-benzaldehyde (6.226g) are dissolved in toluene (190 ml). Subsequently, the mixture is stirred for 16.0 h at reflux in a Dean-Stark apparatus. The reaction mixture is evaporated under reduced pressure, to obtain 9.92 g of (6-chloro-pyridin-3-yl)-[1-(2,4,6-trifluoro-phenyl)-meth-(E)-ylidene]-amine. ¹H NMR (300Mhz, CDCl₃) 8.58ppm, 1 H, s; 8.25ppm, 1H, d, J=2.16Hz; 7.51 ppm, 1H, dd, J=2.47 and 8.36Hz; 7.36ppm, 1 H, d, J=8.39Hz; 6.79ppm, 2H, t, J=8.69Hz.

### b) Preparation 2-Chloro-5-[5-(2,4,6-trifluoro-phenyl)-imidazol-1-yl]-pyridine

9.92 g of (6-Chloro-pyridin-3-yl)-[1-(2,4,6-trifluoro-phenyl)-meth-(E)-ylidene]-amine is dissolved in 120 ml of N,N-dimethyl-formamide and 100 ml of 1,2-dimethoxy-ethane. 10.73 g of toluenesulfonylmethyl isocyanide and 10.13g of anhydrous potassium carbonate are added, and the resulting reaction mixture is heated at 100°C for 90 minutes. After cooling down, the mixture is filtrated, the solvents evaporated, the resulting solid is adsorbed on Isolute^{®} HM-N and purified by chromatography column on silica gel, using a mixture of heptane / ethyl acetate 3:1, 7:3, 2:1 and 1:1 as successive eluents to obtain 9.320 g of an intermediate of molecular weight equal to 465.88 g/mol. This intermediate is dissolved in 80 ml of N,N-dimethyl-formamide and 70 ml of 1,2-dimethoxy-ethane, and to this solution is added 6.645 g of anhydrous potassium carbonate. The resulting reaction mixture is heated at 100°C for 24 h. After cooling down, the mixture is filtrated, the solvents evaporated, the resulting solid is adsorbed on Isolute^{®} HM-N and purified by chromatography column on silica gel, using a mixture of heptane / ethyl acetate 2:1 and 1:1 as successive eluents to obtain 4.782 g of 2-chloro-5-[5-(2,4,6-trifluoro-phenyl)-imidazol-1-yl]-pyridine. ¹H NMR (300Mhz, CDCl₃) 8.26ppm, 1 H, d, J=2.57Hz; 7.81 ppm, 1H, s; 7.48ppm, 1 H, dd, J=2.7 and 8.44Hz; 7.38ppm, 1 H, d, J=8.41Hz; 7.35ppm, 1 H, s; 6.69ppm, 2H, t, J=8Hz.

### c) Preparation of 5-[2-Bromo-5-(2,4,6-trifluoro-phenyl)-imidazol-1-yl]-2-chloro-pyridine

A mixture of 3.290 g of 2-Chloro-5-[5-(2,4,6-trifluoro-phenyl)-imidazol-1-yl]-pyridine, 2.203 g of N-bromosuccinimide and 30 ml of chloroform is heated for 4 h to 80 °C. Subsequently, the mixture is cooled to room temperature, Isolute^{®} HM-N is added to the reaction mixture and the chloroform is evaporated. The crude mixture is purified by chromatography column on silica gel, using a mixture of heptane / ethyl acetate 4:1 as eluent to obtain 1.520 g of 5-[2-bromo-5-(2,4,6-trifluoro-phenyl)-imidazol-1-yl]-2-chloro-pyridine as a pale yellow-orange solid. ¹H NMR (300Mhz, CDCl₃) 8.23ppm, 1 H, d, J=2.58Hz; 7.54ppm, 1 H, dd, J=2.71 and 8.43Hz; 7.40ppm, 1 H, d, J=8.41 Hz; 7.27ppm, 1 H, s; 6.66ppm, 2H, t, J=7.85Hz.

### d) Preparation of 2-Chloro-5-[2-methyl-5-(2,4,6-trifluoro-phenyl)-imidazol-1-yl]-pyridine

5-[2-Bromo-5-(2,4,6-trifluoro-phenyl)-imidazol-1-yl]-2-chloro-pyridine (0.959 g) is dissolved in 50 ml of tetrahydrofurane. To this solution, 0.040 g of tetrakis(triphenylphosphine) Paladium is added, before refluxing the resulting mixture for 10 minutes. After this time, the oil bath is removed and, immediately, 3.7 ml of (trimethyl) Aluminium are added slowly. The reaction mixture is refluxed for exactly 95 minutes before being cooled down to 0°C. 2 ml of methanol are added dropwise (gas evolution) and after five minutes, Isolute^{®} HM-N is added and the solvents removed under reduced pressure. The residue is purified by chromatography on silica gel, using a mixture of heptane / ethyl acetate 1 : 4 as eluent, to deliver 0.218 g of 2-chloro-5-[2-methyl-5-(2,4,6-trifluoro-phenyl)-imidazol-1-yl]-pyridine as a white solid. ¹H NMR (300Mhz, CDCl₃) 8.23ppm, 1 H, d, J=2.53Hz; 7.47ppm, 1 H, dd, J=2.62 and 8.53Hz; 7.39ppm, 1H, d, J=8.4Hz; 7.20ppm, 1 H, s; 6.64ppm, 2H, t, J=7.46Hz; 2.38ppm, 3H, s.

### e) Preparation of 2-Chloro-5-[4-chloro-2-methyl-5-(2,4,6-trifluoro-phenyl)-imidazol-1-yl]-pyridine (Compound No.I.j.210)

A mixture of 0.310 g of 2-chloro-5-[2-methyl-5-(2,4,6-trifluoro-phenyl)-imidazol-1-yl]-pyridine, 0.134 g of N-chlorosuccinimide and 3 ml of chloroform is heated for 3 h to 80 °C. Subsequently, the mixture is cooled to room temperature, Isolute^{®} HM-N is added to the reaction mixture and the chloroform is evaporated. The crude mixture is purified by chromatography column on silica gel, using a mixture of heptane / ethyl acetate 2:1 as eluent to obtain of 2-chloro-5-[4-chloro-2-methyl-5-(2,4,6-trifluoro-phenyl)-imidazol-1-yl]-pyridine. This white solid is dissolved in diethyl ether (10 ml), extracted twice with 5 ml of a 1 N sodium hydroxyde solution, the organic phase is dried over sodium sulfate, filtrated and evaporated under reduced pressure to obtain 0.195 g of 2-chloro-5-[4-chloro-2-methyl-5-(2,4,6-trifluoro-phenyl)-imidazol-1-yl]-pyridine (Compound No.I.j.210) as a white solid. ¹H NMR (300Mhz, CDCl₃) 8.23ppm, 1 H, d, J=2.49Hz; 7.47ppm, 1 H, dd, J=2.63 and 8.43Hz; 7.40ppm, 1 H, d, J=8.41Hz; 6.68ppm, 2H, t, J=7.45Hz; 2.34ppm, 3H, s.

### Example 2: This example illustrates the preparation of 2-Chloro-5-[2,4-dichloro-5-(2,4,6-trifluoro-phenyl)-imidazol-1-yl]-pyridine (Compound No.I.j.209)

A mixture of 0.15 g of 2-chloro-5-[5-(2,4,6-trifluoro-phenyl)-imidazol-1-yl]-pyridine, 0.164 g of N-chlorosuccinimide and 1.56 ml of chloroform is heated for 16 h to 80 °C. Subsequently, the mixture is cooled to room temperature, Isolute^{®} HM-N is added to the reaction mixture and the chloroform is evaporated. The crude mixture is purified by chromatography column on silica gel, using a mixture of heptane / ethyl acetate 6 : 1 as eluent to obtain 0.136 g of 2-chloro-5-[2,4-dichloro-5-(2,4,6-trifluoro-phenyl)-imidazol-1-yl]-pyridine (Compound No.I.j.209). ¹H NMR (300Mhz, CDCl₃) 8.23ppm, 1 H, d, J=2.7Hz; 7.54ppm, 1 H, dd, J=2.7 and 8.5Hz; 7.43ppm, 1 H, d, J=8.36Hz; 6.71 ppm, 2H, t, J=7.97Hz.

Table 1 below illustrates examples of individual compounds of formula I according to the invention.

**Table 1: individual compounds of formula I according to the invention**

| **Compound No.** | **R¹** | **R²** | **R³** |
|---|---|---|---|
| 001 | F | phenyl | F |
| 002 | CH₃ | phenyl | F |
| 003 | F | phenyl | Cl |
| 004 | Cl | phenyl | Cl |
| 005 | CH₃ | phenyl | Cl |
| 006 | F | 3-fluorophenyl | F |
| 007 | CH₃ | 3-fluorophenyl | F |
| 008 | F | 3-fluorophenyl | Cl |
| 009 | Cl | 3-fluorophenyl | Cl |
| 010 | CH₃ | 3-fluorophenyl | Cl |
| 011 | F | 4-fluorophenyl | F |
| 012 | CH₃ | 4-fluorophenyl | F |
| 013 | F | 4-fluorophenyl | Cl |
| 014 | Cl | 4-fluorophenyl | Cl |
| 015 | CH₃ | 4-fluorophenyl | Cl |
| 016 | F | 3-chlorophenyl | F |
| 017 | CH₃ | 3-chlorophenyl | F |
| 018 | F | 3-chlorophenyl | Cl |
| 019 | Cl | 3-chlorophenyl | Cl |
| 020 | CH₃ | 3-chlorophenyl | Cl |
| 021 | F | 4-chlorophenyl | F |
| 022 | CH₃ | 4-chlorophenyl | F |
| 023 | F | 4-chlorophenyl | Cl |
| 024 | Cl | 4-chlorophenyl | Cl |
| 025 | CH₃ | 4-chlorophenyl | Cl |
| 026 | F | 3-bromophenyl | F |
| 027 | CH₃ | 3-bromophenyl | F |
| 028 | F | 3-bromophenyl | Cl |
| 029 | Cl | 3-bromophenyl | Cl |
| 030 | CH₃ | 3-bromophenyl | Cl |
| 031 | F | 4-bromophenyl | F |
| 032 | CH₃ | 4-bromophenyl | F |
| 033 | F | 4-bromophenyl | Cl |
| 034 | Cl | 4-bromophenyl | Cl |
| 035 | CH₃ | 4-bromophenyl | Cl |
| 036 | F | m-tolyl | F |
| 037 | CH₃ | m-tolyl | F |
| 038 | F | m-tolyl | Cl |
| 039 | Cl | m-tolyl | Cl |
| 040 | CH₃ | m-tolyl | Cl |
| 041 | F | p-tolyl | F |
| 042 | CH₃ | p-tolyl | F |
| 043 | F | p-tolyl | Cl |
| 044 | Cl | p-tolyl | Cl |
| 045 | CH₃ | p-tolyl | Cl |
| 046 | F | 3-trifluoromethylphenyl | F |
| 047 | CH₃ | 3-trifluoromethylphenyl | F |
| 048 | F | 3-trifluoromethylphenyl | Cl |
| 049 | Cl | 3-trifluoromethylphenyl | Cl |
| 050 | CH₃ | 3-trifluoromethylphenyl | Cl |
| 051 | F | 4-trifluoromethylphenyl | F |
| 052 | CH₃ | 4-trifluoromethylphenyl | F |
| 053 | F | 4-trifluoromethylphenyl | Cl |
| 054 | Cl | 4-trifluoromethylphenyl | Cl |
| 055 | CH₃ | 4-trifluoromethylphenyl | Cl |
| 056 | F | 3-methoxyphenyl | F |
| 057 | CH₃ | 3-methoxyphenyl | F |
| 058 | F | 3-methoxyphenyl | Cl |
| 059 | Cl | 3-methoxyphenyl | Cl |
| 060 | CH₃ | 3-methoxyphenyl | Cl |
| 061 | F | 4-methoxyphenyl | F |
| 062 | CH₃ | 4-methoxyphenyl | F |
| 063 | F | 4-methoxyphenyl | Cl |
| 064 | Cl | 4-methoxyphenyl | Cl |
| 065 | CH₃ | 4-methoxyphenyl | Cl |
| 066 | F | 3-trifluoromethoxyphenyl | F |
| 067 | CH₃ | 3-trifluoromethoxyphenyl | F |
| 068 | F | 3-trifluoromethoxyphenyl | Cl |
| 069 | Cl | 3-trifluoromethoxyphenyl | Cl |
| 070 | CH₃ | 3-trifluoromethoxyphenyl | Cl |
| 071 | F | 4-trifluoromethoxyphenyl | F |
| 072 | CH₃ | 4-trifluoromethoxyphenyl | F |
| 073 | F | 4-trifluoromethoxyphenyl | Cl |
| 074 | Cl | 4-trifluoromethoxyphenyl | Cl |
| 075 | CH₃ | 4-trifluoromethoxyphenyl | Cl |
| 076 | F | 3-cyanophenyl | F |
| 077 | CH₃ | 3-cyanophenyl | F |
| 078 | F | 3-cyanophenyl | Cl |
| 079 | Cl | 3-cyanophenyl | Cl |
| 080 | CH₃ | 3-cyanophenyl | Cl |
| 081 | F | 4-cyanophenyl | F |
| 082 | CH₃ | 4-cyanophenyl | F |
| 083 | F | 4-cyanophenyl | Cl |
| 084 | Cl | 4-cyanophenyl | Cl |
| 085 | CH₃ | 4-cyanophenyl | Cl |
| 086 | F | 3,4-difluorophenyl | F |
| 087 | CH₃ | 3,4-difluorophenyl | F |
| 088 | F | 3,4-difluorophenyl | Cl |
| 089 | Cl | 3,4-difluorophenyl | Cl |
| 090 | CH₃ | 3,4-difluorophenyl | Cl |
| 091 | F | 3,4-dichlorophenyl | F |
| 092 | CH₃ | 3,4-dichlorophenyl | F |
| 093 | F | 3,4-dichlorophenyl | Cl |
| 094 | Cl | 3,4-dichlorophenyl | Cl |
| 095 | CH₃ | 3,4-dichlorophenyl | Cl |
| 096 | F | 3,4-dimethylphenyl | F |
| 097 | CH₃ | 3,4-dimethylphenyl | F |
| 098 | F | 3,4-dimethylphenyl | Cl |
| 099 | Cl | 3,4-dimethylphenyl | Cl |
| 100 | CH₃ | 3,4-dimethylphenyl | Cl |
| 101 | F | 3,4-dimethoxyphenyl | F |
| 102 | CH₃ | 3,4-dimethoxyphenyl | F |
| 103 | F | 3,4-dimethoxyphenyl | Cl |
| 104 | Cl | 3,4-dimethoxyphenyl | Cl |
| 105 | CH₃ | 3,4-dimethoxyphenyl | Cl |
| 106 | F | 3-chloro-4-fluorophenyl | F |
| 107 | CH₃ | 3-chloro-4-fluorophenyl | F |
| 108 | F | 3-chloro-4-fluorophenyl | Cl |
| 109 | Cl | 3-chloro-4-fluorophenyl | Cl |
| 110 | CH₃ | 3-chloro-4-fluorophenyl | Cl |
| 111 | F | 3-chloro-4-methylphenyl | F |
| 112 | CH₃ | 3-chloro-4-methylphenyl | F |
| 113 | F | 3-chloro-4-methylphenyl | Cl |
| 114 | Cl | 3-chloro-4-methylphenyl | Cl |
| 115 | CH₃ | 3-chloro-4-methylphenyl | Cl |
| 116 | F | 3-chloro-4-methoxyphenyl | F |
| 117 | CH₃ | 3-chloro-4-methoxyphenyl | F |
| 118 | F | 3-chloro-4-methoxyphenyl | Cl |
| 119 | Cl | 3-chloro-4-methoxyphenyl | Cl |
| 120 | CH₃ | 3-chloro-4-methoxyphenyl | Cl |
| 121 | F | 4-chloro-3-fluorophenyl | F |
| 122 | CH₃ | 4-chloro-3-fluorophenyl | F |
| 123 | F | 4-chloro-3-fluorophenyl | Cl |
| 124 | Cl | 4-chloro-3-fluorophenyl | Cl |
| 125 | CH₃ | 4-chloro-3-fluorophenyl | Cl |
| 126 | F | 4-chloro-3-methylphenyl | F |
| 127 | CH₃ | 4-chloro-3-methylphenyl | F |
| 128 | F | 4-chloro-3-methylphenyl | Cl |
| 129 | Cl | 4-chloro-3-methylphenyl | Cl |
| 130 | CH₃ | 4-chloro-3-methylphenyl | Cl |
| 131 | F | 4-chloro-3-methoxyphenyl | F |
| 132 | CH₃ | 4-chloro-3-methoxyphenyl | F |
| 133 | F | 4-chloro-3-methoxyphenyl | Cl |
| 134 | Cl | 4-chloro-3-methoxyphenyl | Cl |
| 135 | CH₃ | 4-chloro-3-methoxyphenyl | Cl |
| 136 | F | 3-fluoro-4-methoxyphenyl | F |
| 137 | CH₃ | 3-fluoro-4-methoxyphenyl | F |
| 138 | F | 3-fluoro-4-methoxyphenyl | Cl |
| 139 | Cl | 3-fluoro-4-methoxyphenyl | Cl |
| 140 | CH₃ | 3-fluoro-4-methoxyphenyl | Cl |
| 141 | F | 3-fluoro-4-methylphenyl | F |
| 142 | CH₃ | 3-fluoro-4-methylphenyl | F |
| 143 | F | 3-fluoro-4-methylphenyl | Cl |
| 144 | Cl | 3-fluoro-4-methylphenyl | Cl |
| 145 | CH₃ | 3-fluoro-4-methylphenyl | Cl |
| 146 | F | 4-fluoro-3-methoxyphenyl | F |
| 147 | CH₃ | 4-fluoro-3-methoxyphenyl | F |
| 148 | F | 4-fluoro-3-methoxyphenyl | Cl |
| 149 | Cl | 4-fluoro-3-methoxyphenyl | Cl |
| 150 | CH₃ | 4-fluoro-3-methoxyphenyl | Cl |
| 151 | F | 4-fluoro-3-methylphenyl | F |
| 152 | CH₃ | 4-fluoro-3-methylphenyl | F |
| 153 | F | 4-fluoro-3-methylphenyl | Cl |
| 154 | Cl | 4-fluoro-3-methylphenyl | Cl |
| 155 | CH₃ | 4-fluoro-3-methylphenyl | Cl |
| 156 | F | 3-methoxy-4-methylphenyl | F |
| 157 | CH₃ | 3-methoxy-4-methylphenyl | F |
| 158 | F | 3-methoxy-4-methylphenyl | Cl |
| 159 | Cl | 3-methoxy-4-methylphenyl | Cl |
| 160 | CH₃ | 3-methoxy-4-methylphenyl | Cl |
| 161 | F | 4-methoxy-3-methylphenyl | F |
| 162 | CH₃ | 4-methoxy-3-methylphenyl | F |
| 163 | F | 4-methoxy-3-methylphenyl | Cl |
| 164 | Cl | 4-methoxy-3-methylphenyl | Cl |
| 165 | CH₃ | 4-methoxy-3-methylphenyl | Cl |
| 166 | F | naphth-2-yl | F |
| 167 | CH₃ | naphth-2-yl | F |
| 168 | F | naphth-2-yl | Cl |
| 169 | Cl | naphth-2-yl | Cl |
| 170 | CH₃ | naphth-2-yl | Cl |
| 171 | F | pyridin-2-yl | F |
| 172 | CH₃ | pyridin-2-yl | F |
| 173 | F | pyridin-2-yl | Cl |
| 174 | Cl | pyridin-2-yl | Cl |
| 175 | CH₃ | pyridin-2-yl | Cl |
| 176 | F | pyridin-3-yl | F |
| 177 | CH₃ | pyridin-3-yl | F |
| 178 | F | pyridin-3-yl | Cl |
| 179 | Cl | pyridin-3-yl | Cl |
| 180 | CH₃ | pyridin-3-yl | Cl |
| 181 | F | pyridin-4-yl | F |
| 182 | CH₃ | pyridin-4-yl | F |
| 183 | F | pyridin-4-yl | Cl |
| 184 | Cl | pyridin-4-yl | Cl |
| 185 | CH₃ | pyridin-4-yl | Cl |
| 186 | F | 6-chloropyridin-2-yl | F |
| 187 | CH₃ | 6-chloropyridin-2-yl | F |
| 188 | F | 6-chloropyridin-2-yl | Cl |
| 189 | Cl | 6-chloropyridin-2-yl | Cl |
| 190 | CH₃ | 6-chloropyridin-2-yl | Cl |
| 191 | F | 6-fluoropyridin-2-yl | F |
| 192 | CH₃ | 6-fluoropyridin-2-yl | F |
| 193 | F | 6-fluoropyridin-2-yl | Cl |
| 194 | Cl | 6-fluoropyridin-2-yl | Cl |
| 195 | CH₃ | 6-fluoropyridin-2-yl | Cl |
| 196 | F | 6-methoxypyridin-2-yl | F |
| 197 | CH₃ | 6-methoxypyridin-2-yl | F |
| 198 | F | 6-methoxypyridin-2-yl | Cl |
| 199 | Cl | 6-methoxypyridin-2-yl | Cl |
| 200 | CH₃ | 6-methoxypyridin-2-yl | Cl |
| 201 | F | 6-methylpyridin-2-yl | F |
| 202 | CH₃ | 6-methylpyridin-2-yl | F |
| 203 | F | 6-methylpyridin-2-yl | Cl |
| 204 | Cl | 6-methylpyridin-2-yl | Cl |
| 205 | CH₃ | 6-methylpyridin-2-yl | Cl |
| 206 | F | 6-chloropyridin-3-yl | F |
| 207 | CH₃ | 6-chloropyridin-3-yl | F |
| 208 | F | 6-chloropyridin-3-yl | Cl |
| 209 | Cl | 6-chloropyridin-3-yl | Cl |
| 210 | CH₃ | 6-chloropyridin-3-yl | Cl |
| 211 | F | 6-fluoropyridin-3-yl | F |
| 212 | CH₃ | 6-fluoropyridin-3-yl | F |
| 213 | F | 6-fluoropyridin-3-yl | Cl |
| 214 | Cl | 6-fluoropyridin-3-yl | Cl |
| 215 | CH₃ | 6-fluoropyridin-3-yl | Cl |
| 216 | F | 6-methoxypyridin-3-yl | F |
| 217 | CH₃ | 6-methoxypyridin-3-yl | F |
| 218 | F | 6-methoxypyridin-3-yl | Cl |
| 219 | Cl | 6-methoxypyridin-3-yl | Cl |
| 220 | CH₃ | 6-methoxypyridin-3-yl | Cl |
| 221 | F | 6-methylpyridin-3-yl | F |
| 222 | CH₃ | 6-methylpyridin-3-yl | F |
| 223 | F | 6-methylpyridin-3-yl | Cl |
| 224 | Cl | 6-methylpyridin-3-yl | Cl |
| 225 | CH₃ | 6-methylpyridin-3-yl | Cl |
| 226 | F | 2-chloropyridin-4-yl | F |
| 227 | CH₃ | 2-chloropyridin-4-yl | F |
| 228 | F | 2-chloropyridin-4-yl | Cl |
| 229 | Cl | 2-chloropyridin-4-yl | Cl |
| 230 | CH₃ | 2-chloropyridin-4-yl | Cl |
| 231 | F | 2-fluoropyridin-4-yl | F |
| 232 | CH₃ | 2-fluoropyridin-4-yl | F |
| 233 | F | 2-fluoropyridin-4-yl | Cl |
| 234 | Cl | 2-fluoropyridin-4-yl | Cl |
| 235 | CH₃ | 2-fluoropyridin-4-yl | Cl |
| 236 | F | 2-methoxypyridin-4-yl | F |
| 237 | CH₃ | 2-methoxypyridin-4-yl | F |
| 238 | F | 2-methoxypyridin-4-yl | Cl |
| 239 | Cl | 2-methoxypyridin-4-yl | Cl |
| 240 | CH₃ | 2-methoxypyridin-4-yl | Cl |
| 241 | F | 2-methylpyridin-4-yl | F |
| 242 | CH₃ | 2-methylpyridin-4-yl | F |
| 243 | F | 2-methylpyridin-4-yl | Cl |
| 244 | Cl | 2-methylpyridin-4-yl | Cl |
| 245 | CH₃ | 2-methylpyridin-4-yl | Cl |
| 246 | F | 5-chlorothiophen-2-yl | F |
| 247 | CH₃ | 5-chlorothiophen-2-yl | F |
| 248 | F | 5-chlorothiophen-2-yl | Cl |
| 249 | Cl | 5-chlorothiophen-2-yl | Cl |
| 250 | CH₃ | 5-chlorothiophen-2-yl | Cl |
| 251 | F | 5-bromothiophen-2-yl | F |
| 252 | CH₃ | 5-bromothiophen-2-yl | F |
| 253 | F | 5-bromothiophen-2-yl | Cl |
| 254 | Cl | 5-bromothiophen-2-yl | Cl |
| 255 | CH₃ | 5-bromothiophen-2-yl | Cl |
| 256 | F | 5-methoxythiophen-2-yl | F |
| 257 | CH₃ | 5-methoxythiophen-2-yl | F |
| 258 | F | 5-methoxythiophen-2-yl | Cl |
| 259 | Cl | 5-methoxythiophen-2-yl | Cl |
| 260 | CH₃ | 5-methoxythiophen-2-yl | Cl |
| 261 | F | quinolin-2-yl | F |
| 262 | CH₃ | quinolin-2-yl | F |
| 263 | F | quinolin-2-yl | Cl |
| 264 | Cl | quinolin-2-yl | Cl |
| 265 | CH₃ | quinolin-2-yl | Cl |
| 266 | F | quinolin-3-yl | F |
| 267 | CH₃ | quinolin-3-yl | F |
| 268 | F | quinolin-3-yl | Cl |
| 269 | Cl | quinolin-3-yl | Cl |
| 270 | CH₃ | quinolin-3-yl | Cl |
| 271 | F | 4-methoxyquinolin-2-yl | F |
| 272 | CH₃ | 4-methoxyquinolin-2-yl | F |
| 273 | F | 4-methoxyquinolin-2-yl | Cl |
| 274 | Cl | 4-methoxyquinolin-2-yl | Cl |
| 275 | CH₃ | 4-methoxyquinolin-2-yl | Cl |
| 276 | F | 4-methylquinolin-2-yl | F |
| 277 | CH₃ | 4-methylquinolin-2-yl | F |
| 278 | F | 4-methylquinolin-2-yl | Cl |
| 279 | Cl | 4-methylquinolin-2-yl | Cl |
| 280 | CH₃ | 4-methylquinolin-2-yl | Cl |

where
a) 280 compounds of formula (I.a): 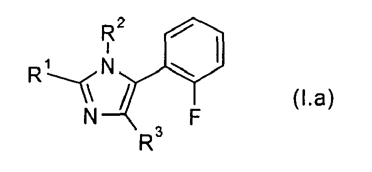 wherein R¹, R² and R³ are as defined in Table 1.
b) 280 compounds of formula (I.b): 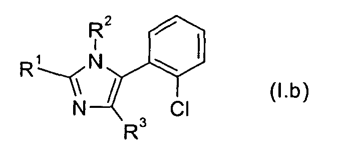 wherein R¹, R² and R³ are as defined in Table 1.
c) 280 compounds of formula (I.c): 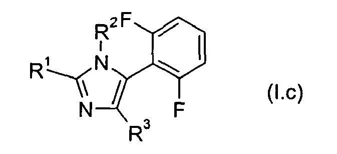 wherein R¹, R² and R³ are as defined in Table 1.
d) 280 compounds of formula (I.d): 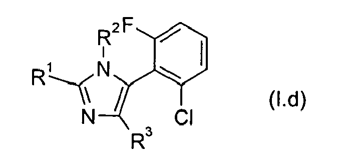 wherein R¹, R² and R³ are as defined in Table 1.
e) 280 compounds of formula (I.e): 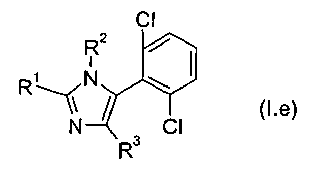 wherein R¹, R² and R³ are as defined in Table 1.
f) 280 compounds of formula (I.f): 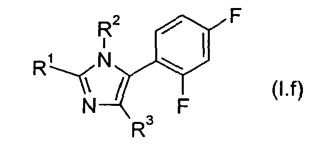 wherein R¹, R² and R³ are as defined in Table 1.
g) 280 compounds of formula (I.g): 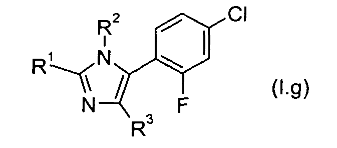 wherein R¹, R² and R³ are as defined in Table 1.
h) 280 compounds of formula (I.h): 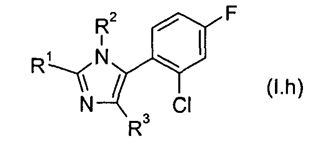 wherein R¹, R² and R³ are as defined in Table 1.
i) 280 compounds of formula (I.i): 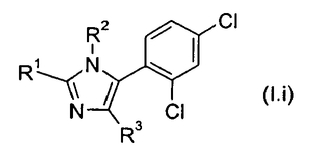 wherein R¹, R² and R³ are as defined in Table 1.
j) 280 compounds of formula (I.j): 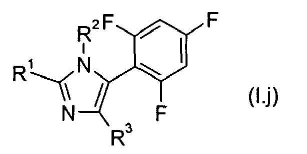 wherein R¹, R² and R³ are as defined in Table 1.
k) 280 compounds of formula (I.k): 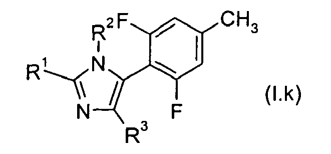 wherein R¹, R² and R³ are as defined in Table 1.
l) 280 compounds of formula (I.l): 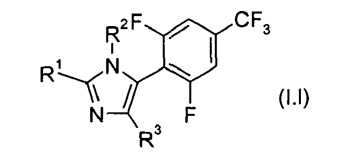 wherein R¹, R² and R³ are as defined in Table 1.
m) 280 compounds of formula (I.m): 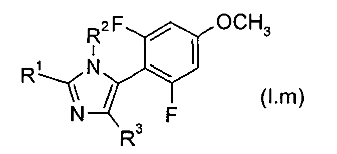 wherein R¹, R² and R³ are as defined in Table 1.
n) 280 compounds of formula (I.n): 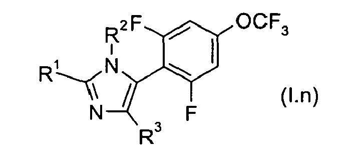 wherein R¹, R² and R³ are as defined in Table 1.
o) 280 compounds of formula (I.o): 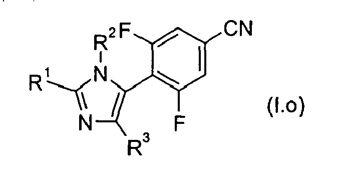 wherein R¹, R² and R³ are as defined in Table 1.
p) 280 compounds of formula (I.p): 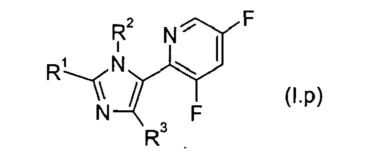 wherein R¹, R² and R³ are as defined in Table 1.
q) 280 compounds of formula (I.q): 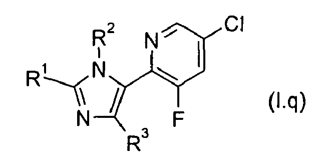 wherein R¹, R² and R³ are as defined in Table 1.
r) 280 compounds of formula (I.r): 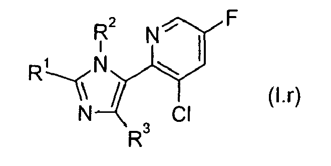 wherein R¹, R² and R³ are as defined in Table 1.
s) 280 compounds of formula (I.s): 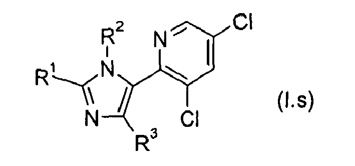 wherein R¹, R² and R³ are as defined in Table 1.
t) 280 compounds of formula (I.t): 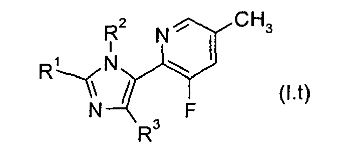 wherein R¹, R² and R³ are as defined in Table 1.
u) 280 compounds of formula (I.u): 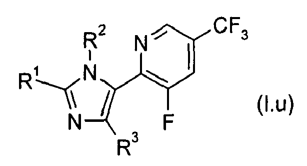 wherein R¹, R² and R³ are as defined in Table 1.
v) 280 compounds of formula (I.v): 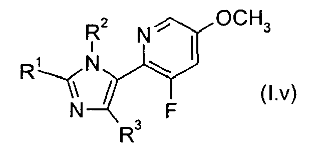 wherein R¹, R² and R³ are as defined in Table 1.
w) 280 compounds of formula (I.w): 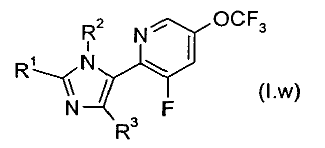 wherein R¹, R² and R³ are as defined in Table 1.
x) 280 compounds of formula (I.x): 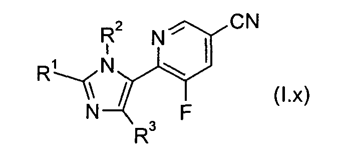 wherein R¹, R² and R³ are as defined in Table 1.
y) 280 compounds of formula (I.y): 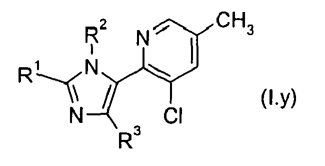 wherein R¹, R² and R³ are as defined in Table 1.
z) 280 compounds of formula (I.z): 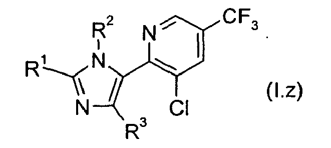 wherein R¹, R² and R³ are as defined in Table 1.

aa) 280 compounds of formula (I.aa): 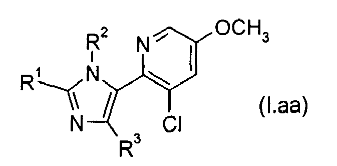 wherein R¹, R² and R³ are as defined in Table 1.
ab) 280 compounds of formula (I.ab): 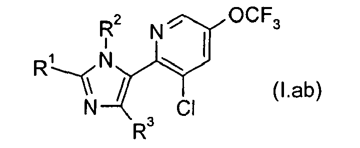 wherein R¹, R² and R³ are as defined in Table 1.
ac) 280 compounds of formula (I.ac): 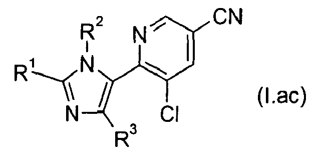 wherein R¹, R² and R³ are as defined in Table 1.

Throughout this description, temperatures are given in degrees Celsius, "m.p." means melting point, "NMR" means nuclear magnetic resonance spectrum; and "%" is percent by weight, unless corresponding concentrations are indicated in other units.

The following abbreviations are used throughout this description:

| | | | |
|---|---|---|---|
| m.p. | = melting point | br | = broad |
| s | = singlet | dd | = doublet of doublets |
| d | = doublet | dt | = doublet of triplets |
| t | = triplet | q | = quartet |
| m | = multiplet | ppm | = parts per million |

Table 2 shows selected NMR data, all with CDCl₃ as the solvent (unless otherwise stated, no attempt is made to list all characterising data in all cases) for compounds of Table 1.

**Table 2: Melting point and selected NMR data for compounds of Table 1**

| Compound Number | ¹H-NMR data (ppm/number of H's/multiplicity) |
|---|---|
| I.j.209 | 8.23ppm, 1H, d, J=2.70Hz; 7.54ppm, 1H, dd, J=2.70 and 8.50Hz; 7.43ppm, 1H, d, J=8.36Hz; 6.71 ppm, 2H, t, J=7.97Hz |
| I.j.210 | 8.23ppm, 1H, d, J=2.49Hz; 7.47ppm, 1H, dd, J=2.63 and 8.43Hz; 7.40ppm, 1H, d, J=8.41 Hz; 6.68ppm, 2H, t, J=7.45Hz; 2.34ppm, 3H, s |
| I.j.225 | 8.32ppm, 1H, d, J=2.26Hz; 7.39ppm, 1H, dd, J=2.41 and 8.21 Hz; 7.21 ppm, 1H, d, J=8.21 Hz; 6.65ppm, 2H, t, J=7.51 Hz; 2.60ppm, 3H, s; 2.32ppm, 3H, s |
| I.j.269 | 8.70ppm, 1H, d, J=2.27Hz; 8.17ppm, 1H, d, J=8.37Hz; 8.06ppm, 1H, s; 7.86ppm, 2H, m; 7.67ppm, 1H, t, J=7.34Hz; 6.64ppm, 2H, t, J=7.82Hz |
| I.j.270 | 8.61 ppm, 1H, d, J=2.24Hz; 8.08ppm, 1H, d, J=8.40Hz; 7.92ppm, 1H, d, J=2.13Hz; 7.77ppm, 1H, d, J=7.54Hz; 7.76ppm, 1H, t, J=8.25Hz; 7.58ppm, 1H, t, J=7.57Hz; 6.52ppm, 2H, t, J=7.79Hz; 2.29ppm, 3H, s |

The compounds according to the present invention can be prepared according to the above-mentioned reaction schemes, in which, unless otherwise stated, the definition of each variable is as defined above for a compound of formula (I).

### Biological examples

### Alternaria solani / tomato / preventive (Action against Alternaria on tomato)

4 weeks old tomato plants cv. Roter Gnom are treated with the formulated test compound in a spray chamber. Two days after application tomato plants are inoculated by spraying a spore suspension on the test plants. After an incubation period of 4 days at 22 °C / 18 °C and 95% r. h. in a greenhouse the disease incidence is assessed.

Compounds of formula I according to the invention, in particular compounds I.j.209, I.j.210, I.j.225, I.j.269 and I.j.270 at 200 ppm inhibit fungal infestation in this test to at least 80 %, while under the same conditions untreated control plants are infected by the phytopathogenic fungi to over 80 %.

### Botrytis cinerea / tomato / preventive (Action against Botrytis on tomato)

4 weeks old tomato plants cv. Roter Gnom are treated with the formulated test compound in a spray chamber. Two days after application tomato plants are inoculated by spraying a spore suspension on the test plants. After an incubation period of 3 days at 20 °C and 95% r. h. in a greenhouse the disease incidence is assessed.

Compounds of formula I according to the invention, in particular compounds I.j.209, I.j.210 and I.j.270 at 200 ppm inhibit fungal infestation in this test to at least 80 %, while under the same conditions untreated control plants are infected by the phytopathogenic fungi to over 80%.

### Puccinia recondita / wheat / preventive (Action against brown rust on wheat)

1 week old wheat plants cv. Arina are treated with the formulated test compound in a spray chamber. One day after application wheat plants are inoculated by spraying a spore suspension (1 x 105 uredospores/ml) on the test plants. After an incubation period of 1 day at 20 °C and 95% r. h. plants are kept for 10 days 20 °C / 18 °C (day/night) and 60% r.h. in a greenhouse. The disease incidence is assessed 11 days after inoculation.

Compounds of formula I according to the invention, in particular compounds I.j.210 and I.j.270 at 200 ppm inhibit fungal infestation in this test to at least 80 %, while under the same conditions untreated control plants are infected by the phytopathogenic fungi to over 80 %.

### Magnaporthe grisea (Pyricularia oryzae) / rice / preventive (Action against rice blast)

3 weeks old rice plants cv. Koshihikari are treated with the formulated test compound in a spray chamber. Two days after application rice plants are inoculated by spraying a spore suspension (1 x 10⁵ conidia/ml) on the test plants. After an incubation period of 6 days at 25°C and 95% r. h. the disease incidence is assessed.

Compounds of formula I according to the invention, in particular compounds I.j.209, I.j.210, I.j.225 and I.j.270 at 200 ppm inhibit fungal infestation in this test to at least 80 %, while under the same conditions untreated control plants are infected by the phytopathogenic fungi to over 80 %.

### Pyrenophora teres (Helminthosporium teres) / barley / preventive (Action against net blotch on barley)

1-week-old barley plants cv. Regina are treated with the formulated test compound in a spray chamber. Two days after application barley plants are inoculated by spraying a spore suspension (2.6 x 10⁴ conidia/ml) on the test plants. After an incubation period of 4 days at 20 °C and 95% r. h. the disease incidence is assessed.

Compounds of formula I according to the invention, in particular compounds I.j.209, I.j.210, I.j.269 and I.j.270 at 200 ppm inhibit fungal infestation in this test to at least 80 %, while under the same conditions untreated control plants are infected by the phytopathogenic fungi to over 80 %.

### Septoria tritici / wheat / preventive (Action against Septoria leaf spot on wheat)

2 weeks old wheat plants cv. Riband are treated with the formulated test compound in a spray chamber. One day after application wheat plants are inoculated by spraying a spore suspension (10⁶ conidia/ml) on the test plants. After an incubation period of 1 day at 22 °C / 21 °C and 95% r. h. plants are kept at 22 °C / 21 °C and 70% r.h. in a greenhouse. The disease incidence is assessed 16 - 18 days after inoculation.

Compounds of formula I according to the invention, in particular compounds I.j.209, I.j.210, I.j.225, I.j.269 and I.j.270 at 200 ppm inhibits fungal infestation in this test to at least 80 %, while under the same conditions untreated control plants are infected by the phytopathogenic fungi to over 80 %.

### Uncinula necator / grape / preventive (Action against powdery mildew on grape)

5 weeks old grape seedlings cv. Gutedel are treated with the formulated test compound in a spray chamber. One day after application grape plants are inoculated by shaking plants infected with grape powdery mildew above the test plants. After an incubation period of 7 days at 24 °C / 22 °C and 70% r. h. under a light regime of 14/10 h (light/dark) the disease incidence is assessed.

Compounds of formula I according to the invention, in particular compounds I.j.210, I.j.225, I.j.269 and I.j.270 at 200 ppm inhibit fungal infestation in this test to at least 80 %, while under the same conditions untreated control plants are infected by the phytopathogenic fungi to over 80 %.

## Claims

1. A compound of formula I: 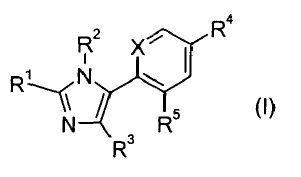 wherein
R¹ is halogen, C₁-C₄alkyl or C₁-C₄haloalkyl;
R² is an optionally substituted aryl or heteroaryl;
R³ is halogen;
R⁴ is hydrogen, halogen, C₁-C₄alkyl, C₁-C₄haloalkyl, C₁-C₄alkoxy, C₁-C₄haloalkoxy or cyano;
R⁵ is halogen;
X is N or C(R); and
R is hydrogen, halogen, C₁-C₄alkyl, C₁-C₄haloalkyl, C₁-C₄alkoxy, C₁-C₄haloalkoxy or cyano; or an agrochemically usable salt form thereof;
provided that
when X is C(R), R² cannot be an optionally substituted aryl.

2. The compound according to claim 1 wherein R¹ is halogen, C₁-C₃alkyl or C₁-C₃haloalkyl.

3. The compound according to either claims 1 or 2 wherein R² is an optionally substituted phenyl, naphthyl, pyridyl, thienyl or quinolyl.

4. The compound according to any one of claims 1 to 3 wherein R³ is fluoro, chloro, bromo or iodo.

5. The compound according to any one of claims 1 to 4 wherein R⁴ is hydrogen, halogen, C₁-C₃alkyl, C₁-C₃haloalkyl, C₁-C₃alkoxy or C₁-C₃haloalkoxy or cyano.

6. The compound according to any one of claims 1 to 5 wherein R⁵ is fluoro, chloro, bromo or iodo.

7. The compound according to any one of claims 1 to 6 wherein X is N, C(H), C(halogen), C(C₁-C₄alkyl), C(C₁-C₄haloalkyl), C(C₁-C₄alkoxy) or C(C₁-C₄haloalkoxy).

8. The compound according to any one of claims 1 to 7 wherein
R¹ is fluoro, chloro, bromo, iodo, C₁-C₂alkyl or C₁-C₂haloalkyl;
R² is an optionally substituted phenyl, pyridyl, thienyl or quinolyl;
R³ is fluoro, chloro or bromo;
R⁴ is hydrogen, fluoro, chloro, bromo, C₁-C₃alkyl, C₁-C₃haloalkyl, C₁-C₃alkoxy, C₁-C₃haloalkoxy or cyano;
R⁵ is fluoro, chloro or bromo; and
X is N, C(H), C(Cl), C(F), C(Br), C(I), C(C₁-C₃alkyl), C(C₁-C₃haloalkyl), C(C₁-C₃alkoxy) or C(C₁-C₃haloalkoxy).

9. The compound according to any one of claims 1 to 8 wherein
R¹ is fluoro, chloro, bromo, methyl or ethyl;
R² is phenyl, 3-fluorophenyl, 4-fluorophenyl, 3-chlorophenyl, 4-chlorophenyl, 3-bromophenyl, 4-bromophenyl, m-tolyl, p-tolyl, 3-trifluoromethylphenyl, 4-trifluoromethylphenyl, 3-methoxyphenyl, 4-methoxyphenyl, 3-trifluoromethoxyphenyl, 4-trifluoromethoxyphenyl, 3-cyanophenyl, 4-cyanophenyl, 3,4-difluorophenyl, 3,4-dichlorophenyl, 3,4-dimethylphenyl, 3,4-dimethoxyphenyl, 3-chloro-4-fluorophenyl, 3-chloro-4-methylphenyl, 3-chloro-4-methoxyphenyl, 4-chloro-3-fluorophenyl, 4-chloro-3-methylphenyl, 4-chloro-3-methoxyphenyl, naphth-2-yl, 3-fluoro-4-methoxyphenyl, 3-fluoro-4-methylphenyl, 4-fluoro-3-methoxyphenyl, 4-fluoro-3-methylphenyl, 3-methoxy-4-methylphenyl, 4-methoxy-3-methylphenyl, pyridin-2-yl, pyridin-3-yl, pyridin-4-yl, 6-chloropyridin-2-yl, 6-fluoropyridin-2-yl, 6-methoxypyridin-2-yl, 6-methylpyridin-2-yl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-methoxypyridin-4-yl, 2-methylpyridin-4-yl, 5-chlorothiophen-2-yl, 5-bromothiophen-2-yl, 5-methoxythiophen-2-yl, quinolin-2-yl, quinolin-3-yl, 4-methoxyquinolin-2-yl or 4-methylquinolin-2-yl;
R³ is fluoro or chloro;
R⁴ is hydrogen, fluoro, chloro, C₁-C₂alkyl, C₁-C₂haloalkyl, C₁-C₂alkoxy, C₁-C₂haloalkoxy or cyano;
R⁵ is fluoro or chloro; and
X is N, C(H), C(Cl), C(F), C(Br), C(C₁-C₂alkyl), C(C₁-C₂haloalkyl), C(C₁-C₂alkoxy) or C(C₁-C₂haloalkoxy).

10. The compound according to any one of claims 1 to 9 wherein
R¹ is fluoro, chloro, methyl or ethyl;
R² is 4-fluorophenyl, 4-chlorophenyl, 4-bromophenyl, p-tolyl, 6-chloropyridin-3-yl, 6-fluoropyridin-3-yl, 6-methoxypyridin-3-yl, 6-methylpyridin-3-yl, 2-chloropyridin-4-yl, 2-fluoropyridin-4-yl, 2-methoxypyridin-4-yl, 2-methylpyridin-4-yl, quinolin-2-yl, quinolin-3-yl, 4-methoxyquinolin-2-yl or 4-methylquinolin-2-yl;
R³ is fluoro or chloro;
R⁴ is hydrogen, fluoro, chloro, C₁-C₂alkyl, C₁-C₂haloalkyl or C₁-C₂alkoxy;
R⁵ is fluoro or chloro; and
X is N, C(H), C(Cl) or C(F).

11. The compound according to any one of claims 1 to 10 wherein
R¹ is chloro or methyl;
R² is 6-chloropyridin-3-yl, 6-methylpyridin-3-yl or quinolin-3-yl;
R³ is chloro;
R⁴ is fluoro; and
R⁵ is fluoro; and
X is C(F).

12. A compound selected from
2-chloro-5-[2,4-dichloro-5-(2,4,6-trifluoro-phenyl)-imidazol-1-yl]-pyridine;
2-chloro-5-[4-chloro-2-methyl-5-(2,4,6-trifluoro-phenyl)-imidazol-1-yl]-pyridine;
5-[4-chloro-2-methyl-5-(2,4,6-trifluoro-phenyl)-imidazol-1-yl]-2-methoxy-pyridine;
2-[4-chloro-2-methyl-5-(2,4,6-trifluoro-phenyl)-imidazol-1-yl]-quinoline;
2-[4-chloro-2-methyl-5-(2,4,6-trifluoro-phenyl)-imidazol-1-yl]-4-methoxy-quinoline;
3-[4-chloro-2-methyl-5-(2,4,6-trifluoro-phenyl)-imidazol-1-yl]-quinoline;
3-[2,4-dichloro-5-(2,4,6-trifluoro-phenyl)-imidazol-1-yl]-quinoline;
3-[2,4-dichloro-5-(2,6-difluoro-4-methoxy-phenyl)-imidazol-1-yl]-quinoline;
2-[4-chloro-5-(2,6-difluoro-4-methoxy-phenyl)-2-methyl-imidazol-1-yl]-4-methoxy-quinoline;
3-[4-chloro-5-(2,6-difluoro-4-methoxy-phenyl)-2-methyl-imidazol-1-yl]-quinoline;
2-chloro-5-[4-chloro-5-(2,6-difluoro-4-methoxy-phenyl)-2-methyl-imidazol-1-yl]-pyridine;
3,5-dichloro-2-[5-chloro-3-(6-chloro-pyridin-3-yl)-2-methyl-3H-imidazol-4-yl]-pyridine; and
2-[4-chloro-5-(3,5-dichloro-pyridin-2-yl)-2-methyl-imidazol-1-yl]-quinoline.

13. A process for the preparation of a compound of formula 1.1, 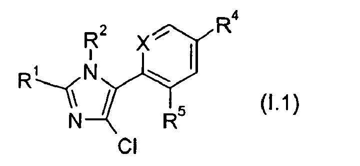 wherein R², R⁴, R⁵ and X are as defined for compound of formula I and R¹ is C₁-C₄alkyl or C₁-C₄haloalkyl, which comprises reacting a compound of formula II, 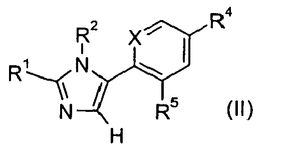 wherein R², R⁴, R⁵ and X are as defined for compound of formula I and R¹ is C₁-C₄alkyl or C₁-C₄haloalkyl, with N-chlorosuccinimide or molecular chlorine.

14. A process for the preparation of a compound of formula II, 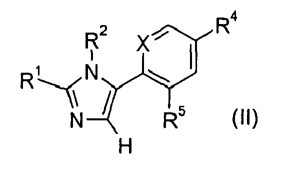 wherein R², R⁴, R⁵ and X are as defined for compound of formula I and R¹ is C₁-C₄alkyl, which comprises reacting a compound of formula III, 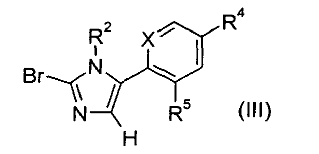 wherein R², R⁴, R⁵ and X are as defined for compound of formula I, with a reagent of formula (R¹)₃Al, wherein R1 is C₁-C₄alkyl in the presence of a transition metal catalyst.

15. A process for the preparation of a compound of formula II, 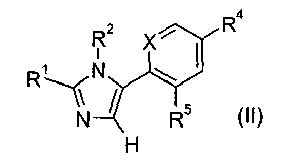 wherein R², R⁴, R⁵ and X are as defined for compound of formula I and R¹ is C₁-C₄alkyl or C₁-C₄haloalkyl, which comprises reacting a compound of formula IV, 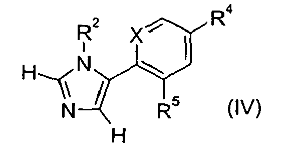 wherein R², R⁴, R⁵ and X are as defined for compound of formula I, with a strong base followed by a reagent of formula R¹Hal, wherein R¹ is C₁-C₄alkyl or C₁-C₄haloalkyl and Hal is halogen.

16. A process for the preparation of a compound of formula I, 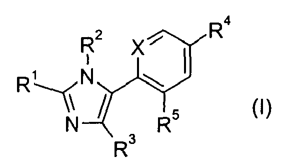 wherein R², R³, R⁴, R⁵ and X are as defined for compound of formula I and R¹ is halogen, which comprises reacting a compound of formula IV, 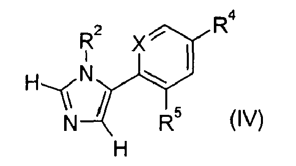 wherein R², R⁴, R⁵ and X are as defined for compound of formula I, with N-chlorosuccinimide, N-bromosuccinimide, molecular chlorine or molecular bromine.

17. A process for the preparation of a compound of formula III, 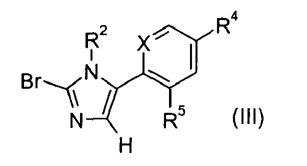 wherein R², R⁴, R⁵ and X are as defined for compound of formula I, which comprises reacting a compound of formula IV, 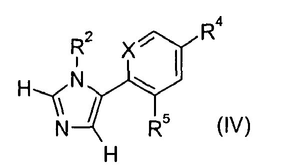 wherein R², R⁴, R⁵ and X are as defined for compound of formula I, with N-bromosuccinimide.

18. A fungicidal composition for controlling or protecting against phytopathogenic microorganisms, comprising as active ingredient at least one compound as defined in any one of claims 1 to 12, in free form or in agrochemically usable salt form, and at least one adjuvant.

19. The composition according to claim 18, which comprises at least one additional fungicidally active compound, preferably selected from the group consisting of azoles, pyrimidinyl carbinoles, 2-amino-pyrimidines, morpholines, anilinopyrimidines, pyrroles, phenylamides, benzimidazoles, dicarboximides, carboxamides, strobilurines, dithiocarbamates, N-halomethylthiotetrahydrophthalimides, copper-compounds, nitrophenols, organo-phosphorus-derivatives, pyridazines, triazolopyrimidines, carboxamides or benzamides.

20. The use of a compound as defined in any one of claims 1 to 12 for controlling or preventing infestation of plants, harvested food crops, seeds or non-living materials by phytopathogenic microorganisms.

21. A method of controlling or preventing an infestation of crop plants, harvested food crops or non-living materials by phytopathogenic or spoilage microorganisms or organisms potentially harmful to man, which comprises the application of a compound as defined in any one of claims 1 to 12, as active ingredient to the plant, to parts of the plants or to the locus thereof, to seeds or to any part of the non-living materials.

22. The method according to claim 21, wherein the phytopathogenic microorganisms are fungal organisms.
